# EUROPEAN PATENT APPLICATION

(11) **EP 1 550 724 A1**
(43) Date of publication of application: **06.07.2005**
(21) Application number: 03807980.2
(22) Date of filing: 30.09.2003
(51) Int. Cl.: C12P 7/62, C08G 63/89

(54) **METHOD OF COAGULATING POLY-3-HYDROXYALKANOIC ACID**

(30) Priority: 30.09.2002 JP 2002285864
(71) Applicant: KANEKA CORPORATION, Osaka-shi, Osaka 530-8288 (JP)
(72) Inventor: OGAWA, Noriko, Kobe-shi, Hyogo 655-0872 (JP); MIYAMOTO, Kenji, Kohokuku, Yokohamashi, Kanagawa 223-0062 (JP); OSAKADA, Fumio, Okayama-shi, Okayama 700-0063 (JP); MATSUMOTO, Keiji, Nishinomiya-shi, Hyogo 663-8023 (JP)
(74) Representative: HOFFMANN - EITLE
(86) International application number: PCT/JP2003/012485
(87) International publication number: WO 2004/033700

(57) **Abstract**

The present invention has for its object to provide a method for agglomerating particles of a poly-3-hydroxyalkanoic acid and enlarging its particle size.

The present invention provides
a method for agglomerating a poly-3-hydroxyalkanoic acid
which comprises suspending particles of the poly-3-hydroxyalkanoic acid in a hydrophilic solvent or a mixture comprising water and a hydrophilic solvent, and
stirring the obtained suspension at a temperature not more than the boiling point of said suspension.

## Description

### TECHNICAL FIELD

The present invention relates to a method for agglomerating particles of a poly-3-hydroxyalkanoic acid.

### BACKGROUND ART

A poly-3-hydroxyalkanoic acid (hereinafter referred to collectively as PHA) is a thermoplastic polyester which is synthesized and accumulated as an energy storage substance in cells of a variety of microorganisms and has biodegradability. In these days waste plastics are disposed of by incineration or landfill but there are several problems in these disposal methods, such as global warming and ground loosening of reclaimed lands. Therefore, with the growing public awareness of the importance of plastics recycling, ways and means for systematized recycling are being developed. However, uses amenable to such recycling are limited. Actually the disposal load of waste plastics cannot be completely liquidated by said incineration, landfill, and recycling but rather a large proportion of the disposal load is not disposed of but simply left in nature. There is accordingly a mounting interest in PHA and other biodegradable plastics which, after disposal, would be incorporated into the natural cycle of matters and degradation products of which would not exert ecologically harmful influences, and their practical utilization are highly desired. Particularly a PHA which a microorganism synthesizes and accumulates in their cells is taken up in the carbon cycle of the natural world and it is, therefore, predicted that it will not have any appreciable adverse effects on the ecosystem. Also in the field of medical treatment, it is considered possible to use a PHA as an implant material which does not require recovery or a vehicle for drug.

Since the PHA synthesized by a microorganism is usually accumulated intracellularly in the form of granules having not more than 1 µm in diameter, exploitation of the PHA as a plastic requires a procedure for separating it from cells. The known technology for the separation and purification of a PHA from microbial cells can be roughly classified into technologies which comprise extracting a PHA from the cells with an organic solvent solving the PHA and the technologies which comprise removing the cell components other than the PHA after cell disruption or solubilization.

Referring to the separation and purification technology of a PHA involving an extraction with an organic solvent, the extraction technique utilizing a halogenated hydrocarbon, such as 1,2-dichloroethane or chloroform, as the solvent solving a PHA is known (refer to Japanese Kokai Publication Sho-55-118394 and Japanese Kokai Publication Sho-57-65193). There also has been proposed an extraction technology using hydrophilic solvents such as dioxane (refer to Japanese Kokai Publication Sho-63-198991), propanediol (refer to Japanese Kokai Publication Hei-02-69187), or tetrahydrofuran (refer to Japanese Kokai Publication Hei-07-79788). However, with such technologies, a solvent layer into which a PHA is extracted is so highly viscous that it involves considerable difficulties in separating the undissolved residues of microbial cells from the PHA-containing solvent layer. In addition, there is a disadvantage that the cost is enormous since the necessary quantity of solvents is so large.

On the other hand, as a technology of removing the cell components other than a PHA by solubilization in a mechanical treatment, a chemical treatment or a catalytic treatment for separation of a PHA, for example, J. Gen. Microbiology, vol. 19, 198-209 (1958) describes a technology which comprises treating a suspension of microbial cells with sodium hypochlorite to solubilize cell components other than a PHA and recovering the PHA. Japanese Kokoku Publication Hei-04-61638 describes a process for separating a PHA which comprises subjecting a suspension of PHA-containing microbial cells to a heat treatment at a temperature of 100°C or higher to disrupt the cellular structure and, then, subjecting the disrupted cells to a combination treatment with a protease and either a phospholipase or hydrogen peroxide to solubilize the cell components other than the PHA.

There also has been proposed a method which comprises treating PHA-containing microbial cells with a surfactant, decomposing the nucleic acids released from the cells with hydrogen peroxide, and separating a PHA (Japanese Kohyo Publication Hei-08-502415).

As a physical treatment method, there has been proposed a technology for separating a PHA which comprises disrupting PHA-containing microbial cells with a high-pressure homogenizer (refer to Japanese Kokai Publication Hei-07-177894 and Japanese Kokai Publication Hei-07-31488).

There has also been proposed a technology for separating a PHA which comprises adding an alkali to a suspension of a PHA-containing microorganism, heating the suspension, and disrupting cells of the microorganism (refer to Japanese Kokai Publication Hei-07-31487). Moreover, several techniques for carrying out physical disruption after addition of an alkali have been proposed (refer to Bioseparation, vol. 2, 95-105, 1991, and Japanese Kokai Publication Hei-07-31489).

There has also been proposed a technology in which a suspension of a PHA-containing microorganism is adjusted to an acidity lower than pH 2 and the PHA is separated by solubilizing cell components other than the PHA at a temperature not below 50°C (Japanese Kokai Publication Hei-11-266891).

The thus-produced PHA is obtained in the form of fine particles having a diameter of not more than 1 µm as it is produced in microbial cells. In many cases, it is more difficult to separate such fine particles from a liquid medium as compared with the case of particles having larger diameter.

Moreover, fine particles are considered to have a risk to cause dust explosion due to their low requiring energy for the explosion and be accumulated in lungs in the case of being aspirated, thus care should be taken for handling.

Therefore, technologies for agglomerating a PHA have been investigated, and a method which comprises agglomeration by heating or an alkaline metal salt, and a method which comprises surfacing and agglomeration, etc. have been developed.

As a technology of agglomeration by heating, there is a method which comprises heating a suspension containing poly-3-hydroxybutyrate (hereinafter, referred to as PHB) to the vicinity of the melting point of PHB to agglomerate (refer to Bailey, Neil A.; George, Neil; Niranjan, K.; Varley, Julie. Biochemical Engineering group, University Reading, "IchemE Res. Event, Eur. Conf. Young Res. Chem. Eng." (United Kingdom), second edition, Institution of Chemical Engineers, 1996, vol.1, 196-198). Japanese Kohyo Publication Hei-07-509131 proposes a technology which comprises directly injecting vapor having an appropriate temperature and pressure to a copolymer of PHB suspended in water and D-3-hydroxyvalerate (3HV) (hereinafter, referred to as PHBV), then heating and stirring at 120 to 160°c to enlarge the particle size of PHBV. These technologies require processes of injecting vapor which is heated and pressurized, and heating to a very high temperature. Therefore, a special equipment capable of high-temperature heating and incubation, and further having pressure-resistance is required. Moreover, there is a possibility of causing the decrease in molecular weight since the treatment is carried out at considerably high temperature.

Japanese Kokai Publication Hei-04-264125 proposes a technology of recovering PHB after precipitating PHB in the form of floc, which comprises extracting PHB from PHB-containing cells in organic solvents, which are not miscible with water and has the boiling point of below 100°C such as methylene chloride, chloroform and trichloroethylene, under water-containing condition while heated and stirred, and pouring said organic phase containing the extracted PHB into hot water. This technology is one of crystallizing technologies of PHB, but does not agglomerate PHB substantially. Additionally, this technology comprises very complicated processes, therefore has difficulties for an industrial application. Moreover, 10 to 30 times weight of the organic solvent relative to that of dried microbial cell is required. Furthermore, since the use of organohalogen compounds tends to be limited for protection of the environment these days, they are not desirable to be used.

As a technology of agglomerating a PHA by adding an alkali metal salt, there has been known an agglomeration method using a bivalent cation (refer to J. Biotechnol., 1998, vol.65(2, 3), 173-182). Particularly, there has been reported a technology of separating PHB by adding calcium chloride, magnesium sulfate, magnesium chloride and magnesium acetate to a PHB suspension to agglomerate PHB (Japanese Kohyo Publication Hei-05-507410). However, this technology makes metal salts mixed into a polymer, therefore is not preferable depending on the products.

### SUMMARY OF THE INVENTION

The object of the present invention is to overcome the above disadvantages of the prior art and accordingly provide a technology of obtaining a PHA aggregate with high purity and handling easiness while inhibiting the decrease in molecular weight.

The inventors of the present invention explored in earnest for obtaining a PHA aggregate with advantage commercially. As a result, they found that PHA particles are agglomerated by suspending fine PHA particles in a hydrophilic solvent or a mixture comprising water and a hydrophilic solvent, and stirring the obtained suspension at a temperature of not more than the boiling point of said suspension, to thereby obtain PHA aggregate with high purity and excellent in filterability and operability. Thus the present invention has been completed.

The present invention comprises suspending PHA particles in a hydrophilic solvent or a mixture comprising water and a hydrophilic solvent, and stirring, to agglomerate the particles. The temperature for agglomerating the PHA suspended in a hydrophilic solvent or a mixture comprising water and a hydrophilic solvent is not more than the boiling point of said suspension, but for obtaining sufficiently agglomerated PHA more efficiently, preferably the suspension is incubated and stirred at the boiling point of said suspension. According to the agglomeration method of the present invention, since impurities contained in a PHA (e.g. lipid) can be solved and removed, the purity of the PHA can be enhanced. Moreover, in the method of the present invention, conditions such as high-temperature and high-pressure that require a special equipment are not necessarily needed.

### DETAILED DESCRIPTION OF THE INVENTION

The term "PHA" as used in this specification is a generic term meaning any and all polymers of hydroxyalkanoic acids. Although the hydroxyalkanoic acid units of such polymers are not particularly restricted, a homopolymer of D-3-hydroxybutyrate (hereinafter, referred to as 3HB), a copolymer of 3HB and one or more other 3-hydroxyalkanoic acids, and a copolymer of D-3-hydroxyhexanoate (hereinafter, referred to as 3HH) and one or more other D-3-hydroxyalkanoic acids may be mentioned by way of example. Additionally, there may be mentioned a copolymer constituted from at least two species of monomers selected from the group consisting of 3-hydroxypropionate, 3-hydroxybutyrate, 3-hydroxyvalerate, 3-hydroxyhexanoate, 3-hydroxyheptanoate and 3-hydroxyoctanoate. Particularly preferred from the standpoint of characteristics of the product polyester is the polymer containing 3HH as a monomeric unit, for example a binary copolymer comprising 3HB and 3HH (PHBH) (Macromolecules, 28, 4822-4828 (1995)) or a ternary copolymer comprising 3HB, D-3-hydroxyvalerate (hereinafter, referred to as 3HV), and 3HH (PHBVH) (Japanese Patent No. 277757, Japanese Kokai Publication Hei-08-289797). The compositional ratio of the monomer units constituting a binary copolymer comprising 3HB and 3HH is not particularly restricted but copolymers containing 1 to 99 mol % of the 3HH unit are suitable. The compositional ratio of the monomer units constituting a ternary copolymer PHBVH comprising 3HB, 3HV and 3HH is not particularly restricted either, but copolymers containing 1 to 95 mol % of the 3HB unit, 1 to 96 mol % of the 3HV unit, and 1 to 30 mol % of the 3HH unit are preferred.

The hydrophilic solvent used in the present invention is not particularly restricted, and may be alcohols such as methanol, ethanol, 1-propanol, 2-propanol, 1-butanol, 2-butanol, iso-butanol, pentanol, hexanol, hepatanol; ketones such as acetone and methylethylketone; ethers such as tetrahydrofuran and dioxane; nitriles such as acetonitrile and propionitrille; amides such as dimethylformamide and acetoamide; dimethylsulfoxide, pyridine, piperidine, and the like. Preferred among them are methanol, ethanol, 1-propanol, 2-propanol, 1-butanol, 2-butanol, iso-butanol, acetone, methylethylketone, tetrahydrofuran, dioxane, acetonitrile and propionitrille from the standpoint of removability. Methanol, ethanol, 1-propanol, 2-propanol, butanol and acetone are more preferred from the point of their ready availability. Still more preferred are methanol, ethanol and acetone.

The concentration of a PHA in the suspension is not particularly restricted, but preferably not less than 1 g/L, more preferably not less than 10 g/L, and still more preferably not less than 30 g/L. Furthermore, it is preferably not more than 500 g/L, more preferably not more than 300 g/L, and still more preferably not more than 200 g/L. If the concentration of a PHA is extremely high, the viscosity of the suspension becomes increased, thus the suspension tends to be substantially non-fluid.

The medium of the suspension may be composed of a hydrophilic solvent solely, or of a mixture comprising water and a hydrophilic solvent. The concentration of the hydrophilic solvent in the mixture is not particularly restricted provided that it is not more than the solubility of the hydrophilic solvent to be used to water, but preferably not less than 10% v/v, and more preferably not less than 20% v/v for obtaining more sufficient agglomeration effect.

In the agglomeration method of the present invention, PHA particles are agglomerated by stirring a suspension obtained by suspending the PHA particles in a hydrophilic solvent or a mixture comprising water and a hydrophilic solvent under the boiling point of said suspension. Stirring means is not particularly restricted and includes a stirring tank, etc. which causes turbulent flow.

The temperature at the time of stirring is preferably not less than room temperature, more preferably not less than 40°C, and still more preferably not less than 60°C. But from the standpoint of agglomeration efficiency, it is more preferable to be nearer to the boiling point of the suspension, and most preferably, the boiling point of the suspension. In the specification, "the boiling point of a suspension" means the temperature that the suspension begins to boil. In the method of the invention, PHA particles may be generally agglomerated at a temperature of not more than 100°C. Moreover, the agglomeration method of the present invention may be carried out under normal pressure, with no necessity of pressurizing, although pressurized condition may also be applied.

Period of time required for agglomeration differs depending on conditions such as the temperature or the concentration, generally the particles are agglomerated sufficiently in several minutes to several hours.

According to the agglomeration method of the present invention, it becomes possible to enlarge the particle size of PHA aggregates. For example, aggregates having the volume average diameter of not less than 20 µm, preferably not less than 50 µm, and more preferably not less than 100 µm can be obtained. The upper limit thereof is not particularly restricted, but aggregates having the volume average diameter of not more than 1000 µm, and preferably not less than 500 µm may be obtained. As the particle size increases, recovery by filtration is made easy, thus equipment cost for industrial productions may be reduced.

The agglomeration method of the present invention may preferably be applied to a PHA obtained by separation and purification of the PHA produced by microorganisms from said microbial cells. In this case, cell constituent substances surrounding the particles are required to be decomposed at a sufficient extent that at least PHA particles are contacted each other in the suspension.

In the case that the PHA particles recovered at the first is contaminated with substances other than the PHA such as soluble cell constituent substances and decomposition products, etc., it is possible, in particular, to suspend those again in the second liquid medium and subject the mixture to successive processes such as washing by stirring and a chemical treatment (e. g. treatment using bleach such as hydrogen peroxide or sodium hypochlorite), and recover the particles from the new liquid medium. The agglomeration method of the present invention may be carried out at any points in this process.

Said microorganism is not particularly restricted provided that it is a microorganism containing a PHA as intracellularly accumulated. For example, microorganisms of the genus Alcaligenes; those of the genus Ralstonia; those of the genus Pseudomonas; those of the genus Bacillus, those of the genus Azotobacter; those of the genus Nocardia; and those of the genus Aeromonas may be mentioned. Among them, preferred are strains of A. lipolytica, A. latus, A. caviae, A. hydrophila and R. eutropha, further preferably are R. eutropha transformed by a gene of a PHA synthase group derived from A. caviae (old name: Alcaligenes eutrophus AC32 (deposited on Budapest Treaty, international depositary authority: National Institute of Advanced Industrial Science and Technology International Patent Organism Depositary, Chuo 6, 1 Higashi 1 chome, Tsukuba-shi, Ibaraki-ken, Japan, date of deposit: August 7, 1997, Deposition No. FERM BP-6038, as transferred from FERM P-15786 originally deposited (J. Bacteriol., 179, 4821-4830 (1997)). Cells, in which a PHA is accumulated intercellularly by culturing in a suitable condition, are used. The cultural method is not particularly restricted but the known method described in Japanese Kokai Publication Hei-05-93049, among others, may for example be employed.

As the PHA particle for use in the agglomeration method according to the present invention, there may be used optional PHA particles obtained from PHA-containing microbial cells by well-known methods described in the chapter of Background Art. Preferable methods for separating the PHA particle from PHA-containing microbial cells include a method comprising, while stirring a suspension of PHA-containing microbial cells, solubilizing cell constituent substances other than the PHA to separate the PHA by adding an alkali simultaneously with physical disruption. By this method, it becomes possible to obtain aggregates of PHA particles from PHA-containing microbial cells with a very simple process and also in high efficiency.

The term "a suspension of microbial cells" means a culture suspension after completion of culture as such, or an aqueous suspension in which microbial cells separated from culture medium by centrifugation, etc. is suspended in water. The concentration for the suspension of cells is preferably not more than 500 g/L, and more preferably not more than 300 g/L in terms of dried microbial cells.

The physical disruption treatment is not particularly restricted provided that it is capable of disrupting nucleic acid efficiently, which is solubilized from cells by an alkaline treatment and becomes a main cause of the increase in viscosity as well as capable of dispersing insoluble substances other than polymers, such as cell wall, cell membrane and insoluble protein. Specifically, there may be mentioned, not only disruption by sonication but also disruption with an emulsification-dispersion machine, a high-pressure homogenizer, a mill or the like.

The alkali is not particularly restricted and may be alkali metals or hydroxides of an alkaline earth metal such as sodium hydroxide, potassium hydroxide, lithium hydroxide and calcium hydroxide; alkali metal carbonates such as sodium carbonate and potassium carbonate; alkali metal salts of organic acids, such as sodium acetate and potassium acetate; alkali metal borates such as borax etc.; alkali metal phosphates such as trisodium phosphate, disodium hydrogen phosphate, tripotassium phosphate and dipotassium hydrogen phosphate, and aqueous ammonia, among others. Among these, sodium hydroxide, sodium carbonate and potassium hydroxide are preferred in terms of suitability for commercial production and in cost terms.

When adding the alkali, it is preferable to control the pH of the suspension by said alkali addition. Particularly, the control target pH value is preferably within a range of pH 8 to 13.5, more preferably pH 10 to 13, still more preferably pH 11 to 13. For controlling the pH, the alkali is preferably added either continuously or intermittently with measuring the pH of the suspension.

The temperature for carrying out the physical disruption and the alkali treatment is not particularly restricted, but preferably between at a room temperature and 50°C, more preferably between at 30°C and 40°C.

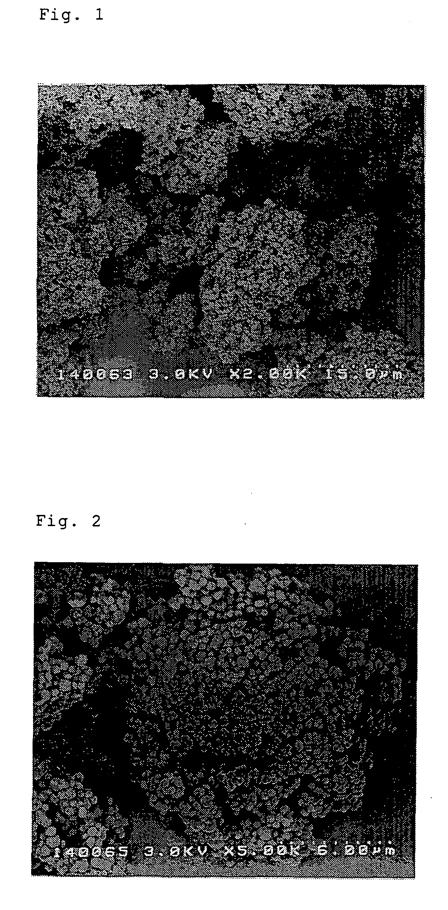

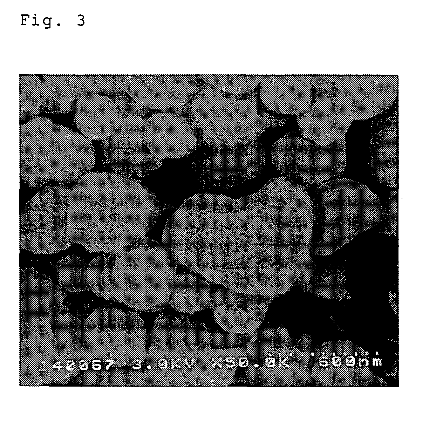

Fig. 1 is a photograph of PHA aggregates according to one embodiment of the present invention taken by a scanning electron microscope (SEM). It is found that one aggregate is formed by adhesion among many PHBH microparticles having a particle diameter between about 0.1 µm and about 1.5 µm. These diameters of microparticles were determined by a method known for the person skilled in the art using a scanning electron microscope. Namely, particle diameters of the microparticles were determined by, at first, photographing a surface image of a PHA aggregate in five thousandfold resolution, and reading the diameter of individual microparticle directly from the obtained photograph.

### BRIEF DESCRIPTION OF THE DRAWINGS

Fig.1 is a scanning electron microscope photograph (x 2,000) of PHBH agglomerates.
Fig.2 is a scanning electron microscope photograph (x 5,000) of a single PHBH agglomerate.
Fig.3 is a scanning electron microscope photograph (x 50,000) of a PHBH agglomerate.

### BEST MODE FOR CARRYING OUT THE INVENTION

In the examples, PHBH was used as a PHA. The embodiments of the present invention are by no means limited to PHBH.

A suspension of PHBH was obtained by culturing R. eutropha (deposit number FERM BP-6038) transformed by a gene in the PHA synthase group derived from Aeromonas caviae in accordance with the protocol given in J. Bacteriol., 179, 4821-4830 (1997) to harvest bacterial cells containing about 67 wt % of PHBH. The pasty cellular fraction separated from the culture medium thus obtained by centrifugation (5, 000 rpm, 10 min) was diluted with water to prepare an aqueous suspension of 75 g dried cells/L concentration. Cell constituent substances other than PHBH were solubilized by stirring and disrupting physically while maintaining the pH at 11.7 by adding an aqueous solution of sodium hydroxide as an alkali, and a precipitate was obtained by centrifugation (3,000 rpm, 10 min). The precipitate was further washed with water to separate PHBH having an average molecular weight of approximately 1, 400, 000, 3HH mole fraction of 7%, and purity of 97%. The thus-obtained PHBH was used in the following experiments as an aqueous suspension of 20% w/v in concentration.

The purity of PHBH used in respective Examples and Comparative Examples was determined as follows. (However, in Examples 3 and 4, the purity was determined by HPLC method described hereinafter.) 10 mg of PHBH powder was dissolved in 1 ml of chloroform and treated with 0.85 ml of methanol and 0.15 ml of concentrated sulfuric acid at 100°C for 140 minutes. After cooling, 0.5 ml of a saturated aqueous solution of ammonium sulfate was added, the mixture was stirred vigorously and, then, allowed to stand. The bottom layer was analyzed by capillary gas chromatography to determine the purity of PHBH in the separated substance and mole fraction of 3HB and 3HH in PHBH.

The molecular weight of the PHBH separated from the bacterial cell was determined as follows. 10 mg of the precipitate separated from the bacterial cells was dissolved in 1 ml of chloroform and the solution was filtered to remove the insoluble substance. The filtrate was analyzed with SHIMADZU's GPC System fitted with Shodex K805L (300 x 8 mm, two columns connected in series) using chloroform as the mobile phase.

The diameter of PHA particle was measured by using Microtrac particle size analyzer manufactured by NIKKISO CO., Ltd, and obtained as a volume average diameter. The volume average diameter is generally used to express a particle diameter, and means an average particle diameter weighed by particle volume.

### (Example 1)

40 ml of a 20% w/v PHBH aqueous suspension and 160 ml of ethanol were mixed, and the mixture was heated and stirred for 10 minutes in a stirring tank with a bath temperature of 90°C. Then, the obtained mixture was cooled to room temperature with stirring. A polymer was recovered by centrifugation (2,400 rpm, 15 min), and measured for the particle diameter after washed with water. The result is shown in Table 1.

**Table 1**

| | Particle diameter (µm) | Molecular weight ×10⁴ | Purity (%) |
|---|---|---|---|
| Non-treated | 8.6 | 144 | 97 |
| Treated | 199 | 130 | 97 |

From the result, it was found that when the suspension comprising a mixture of water and a hydrophilic solvent was heated and stirred, PHA particles were agglomerated, thereby the particle diameter was increased without significant decrease of the molecular weight of PHA.

### (Example 2)

25 ml of the PHBH suspension same as that used in Example 1 was added with various hydrophilic solvents, and stirred for 15 minutes at a bath temperature of 80°C. Thereafter, the mixture was cooled to room temperature with stirring, and PHA was recovered by centrifugation (2,400 rpm, 150 min). The obtained PHA was washed with water and resuspended in water to measure the particle diameter.

**Table 2**

| Organic solvent(ml) | Particle diameter (µm) | Molecular weight ×10⁴ |
|---|---|---|
| Methanol(75) | 201 | 141 |
| Acetone(25) | 29 | 137 |
| Acetone(75) | >1000 | 131 |
| Acetonitrile(25) | >1000 | 132 |
| Tetrahydrofuran (25) | >1000 | 127 |

From the result, it was found that when a suspension comprising a mixture of water and hydrophilic solvent was heated and stirred, PHA particles were agglomerated, thereby particle diameter was increased without significant decrease of the molecular weight of PHA. Particularly, when the solvents having high PHA solubility such as acetonitrile and tetrahydrofuran were used, the particle diameter became more increased.

### (Example 3)

Using the PHBH slurry used in Example 1, PHBH aqueous suspensions were prepared in such a manner that the content of ethanol was to be 80 mL and 70 mL in 100 mL of a suspension containing 10 g of PHBH (pH of the respective suspensions was 7.62 and 7.36), the suspensions were heated and stirred in a stirring tank with a bath temperature of 90°C. Samples were taken from the suspension at an appropriate time, and were cooled to room temperature with stirring. The samples were further recovered by centrifugation (2,400 rpm, 15 min), resuspended in water, and measured for the particle diameter. The results are shown in Table 3.

**Table 3**

| Content of ethanol (mL) | Heating time (min) | Particle diameter (µm) | Molecular weight ×10⁴ | Purity(%) |
|---|---|---|---|---|
| 80 | 0 | 8.6 | 144 | 97 |
| | 10 | 123 | 144 | 98.6 |
| | 15 | 211 | 135 | >99 |
| 70 | 0 | 8.6 | 144 | 97 |
| | 10 | 140 | 142 | 98.9 |
| | 15 | 118 | 140 | >99 |

From the result, it was found that when the suspension comprising a mixture of water and a hydrophilic solvent was heated and stirred, PHA particles were agglomerated without significant decrease of the molecular weight of the PHA, and the particle diameter was increased. Moreover, the purity of the PHA was also found to be improved.

### (Example 4)

PHBH (molecular weight: 1,560,000, purity 99%) separated from microbial cells in the same manner as Example 1 was heated and dried under reduced pressure. 8 g of the resultant dried PHBH was suspended sufficiently in ethanol to obtain 80 mL of a PHBH ethanol suspension (pH 7.05). The suspension was heated and stirred in the same manner as Example 1, cooled, and suspended in water to be measured for the particle diameter. The results are shown in Table 4.

**Table 4**

| Heating time (min) | Particle diameter (µm) | Molecular weight ×10⁴ | Purity |
|---|---|---|---|
| 0 | 24 | 156 | 98.8 |
| 10 | 92 | 156 | 99.1 |
| 15 | 125 | 154 | >99 |

From the result, it was found that when a suspension comprising a hydrophilic solvent was heated and stirred, PHA particles were agglomerated without causing the decrease in molecular weight, and the particle diameter was increased. Moreover, the purity of PHA was found to be improved.

In Examples 3 and 4, the purity of the treated PHBH was determined as follows.

The treated PHBH suspension was centrifuged to remove a supernatant, and the recovered PHBH was washed with ethanol twice in such a manner that ethanol was added until the amount of the suspension became equal to the amount of said PHBH suspension. After the washing, the purity of PHBH dried by heating (50°C) under reduced pressure was determined using high-performance liquid chromatography.

Conditions of the high-performance liquid chromatography:
Column; Shiseido CAPCELL PAK UG 80 4.6 mm x 250 mm
Mobile phase; 20 mmol Phosphate buffer(pH 3.0): methanol = 80:20 (adjusted by potassium phosphate + phosphoric acid)
Flow rate; 1.0 mL/min
Column temperature; 40°C

Approximately 25 mg of polymer, 4 mL of methanol, and 300 µL of methanesulfonic acid were mixed, heated at 100°C for 3 hours, then the mixture was cooled to room temperature, and was messed up with 10 mL of methanol. 10 µL of the obtained mixture was injected into the high-performance liquid chromatography.

### (Example 5)

The aggregates obtained by the methanol agglomeration in Example 2 were photographed with a scanning electron microscope (Figs. 1 to 3). The aggregates were sampled by a scattering method and as a result of an observation with HITACHI S-4000 scanning electron microscope in an accelerating voltage of 3 kV, it was found that roundish PHBH particles in submicron order are agglomerated to form amorphous secondary aggregates (Figs. 1 and 2). Furthermore, as a result of an observation with HITACHI S-5000 scanning electron microscope in an accelerating voltage of 1 kV, it was made clear that there were a portion in which particles are jointed each other (Fig. 3).

### INDUSTRIAL APPLICABILITY

The method for agglomerating a PHA according to the present invention makes it possible to produce PHA aggregates with high purity while inhibiting the decrease in molecular weight with a very simple process. By this method, the PHA becomes a particle having a diameter which is excellent in filterability and operability.

## Claims

1. A method for agglomerating a poly-3-hydroxyalkanoic acid suspended in liquid mixture
which comprises suspending particles of the poly-3-hydroxyalkanoic acid in a hydrophilic solvent or a mixture comprising water and a hydrophilic solvent, and
stirring the obtained suspension at a temperature not more than the boiling point of said suspension.

2. The method according to Claim 1,
wherein the poly-3-hydroxyalkanoic acid is a copolymer constituted of at least two species of monomers selected from the group consisting of 3-hydroxypropionate, 3-hydroxybutyrate, 3-hydroxyvalerate, 3-hydroxyhexanoate, 3-hydroxyheptanoate and 3-hydroxyoctanoate.

3. The method according to Claim 1,
wherein the poly-3-hydroxyalkanoic acid is a copolymer derived from D-3-hydroxyhexanoate and one or more other D-3-hydroxyalkanoic acids.

4. The method according to Claim 3,
wherein the poly-3-hydroxyalkanoic acid is a binary copolymer derived from D-3-hydroxyhexanoate and D-3-hydroxybutyrate or a ternary copolymer derived from D-3-hydroxyhexanoate, D-3-hydroxybutyrate and D-3-hydroxyvalerate.

5. The method according to any one of Claims 1 to 4,
wherein the poly-3-hydroxyalkanoic acid is produced by a microorganism, and separated and purified from said microorganism.

6. The method according to Claim 5,
wherein the microorganism producing the poly-3-hydroxyalkanoic acid belongs to the genus Aeromonas.

7. The method according to Claim 6,
wherein the microorganism producing the poly-3-hydroxyalkanoic acid is Aeromonas caviae or Aeromonas hydrophila.

8. The method according to Claim 5,
wherein the microorganism producing the poly-3-hydroxyalkanoic acid is a cell transformed by a gene in the poly-3-hydroxyalkanoic acid synthase group, derived from Aeromonas caviae.

9. The method according to Claim 5,
wherein the microorganism containing a poly-3-hydroxyalkanoic acid is Ralstonia eutropha transformed by a gene in the poly-3-hydroxyalkanoic acid synthase group, derived from Aeromonas caviae.

10. The method according to any one of Claims 1 to 9,
wherein the particle of the poly-3-hydroxyalkanoic acid is obtainable by, while stirring a suspension of a poly-3-hydroxy alkanoic acid-containing microbial cells,
solubilizing cell constituent substances other than the poly-3-hydroxyalkanoic acid by adding an alkali simultaneously with physical disruption, to separate the poly-3-hydroxyalkanoic acid.

11. The method according to any one of Claims 1 to 10,
wherein the hydrophilic solvent is one selected from the group consisting of alcohols, ketones, nitriles, amides and ethers.

12. The method according to Claim 11,
wherein the alcohol is methanol or ethanol, the ketone is acetone, the nitrile is acetonitrile, the amide is dimethylformamide, and the ether is tetrahydrofuran.

13. An aggregate of poly-3-hydroxyalkanoic acids
which is formable by adhesion among poly-3-hydroxyalkanoic acid microparticles having a particle diameter of at least 0.1 µm and at most 1.5 µm.
